# EUROPEAN PATENT SPECIFICATION

(11) **EP 1 337 262 B1**
(45) Date of publication and mention of the grant of the patent: **27.08.2008**
(21) Application number: 01987313.2
(22) Date of filing: 08.11.2001
(51) Int. Cl.: A61K 33/40, A61L 12/14, A61L 12/12, A61L 12/08, A61K 9/00

(54) **IMPROVED OPHTHALMIC AND CONTACT LENS SOLUTIONS WITH A PEROXIDE SOURCE AND A CATIONIC POLYMERIC PRESERVATIVE**
VERBESSERTE OPHTHALMOLOGISCHE UND KONTAKTLINSENLÖSUNGEN MIT EINER PEROXIDQUELLE UND EINEM KATIONISCHEN POLYMEREN KONSERVIERUNGSMITTEL
SOLUTIONS OPHTALMIQUES ET POUR VERRES DE CONTACT AMELIOREES A SOURCE DE PEROXYDE ET CONSERVATEUR CATIONIQUE POLYMERE

(30) Priority: 08.11.2000 US 246689 P; 08.11.2000 US 246707 P; 08.11.2000 US 246708 P; 08.11.2000 US 246709 P
(43) Date of publication of application: 27.08.2003
(73) Proprietor: FXS Ventures, LLC, Salem, NH 03079 (US)
(72) Inventor: SMITH, Francis, Xavier, Salem, NH 03079 (US)
(74) Representative: Wagner, Karl H.
(86) International application number: PCT/US2001/046882
(87) International publication number: WO 2002/040062

(56) References cited:
- WO-A-92/11876
- WO-A-97/34834
- US-A- 4 891 423
- US-A- 5 449 658
- US-A- 5 718 895

## Description

### Background

This invention relates to ophthalmic solutions used to treat eyes, to deliver active pharmaceutical agents to eyes and to treat ophthalmic devices that in use directly contact corneal tissues. Ophthalmic solutions are used to regularly treat and condition eyes and articles and devices that are regularly used in eyes, such as contact lenses. Because of the intimate contact that such solutions have with corneal tissue, several problems or concerns are regularly presented. For instance, for solutions directly in contact with corneal tissue the compatibility of the solution with the tissue, its ability to not damage or irritate, is important. This compatibility issue is also important for solutions used to treat devices that contact corneal tissue, such as contact lenses and the like. Furthermore, prolonged contact with corneal tissue can lead to the accumulation of material on corneal tissue, or on devices in contact with the solution that then leads to adverse reactions.

Preservative efficacy is measured by the amount that a solution decreases the viability of bacterial or fungal populations. In general, there is an expected trade-off between preservative efficacy and corneal tissue compatibility, as well as "comfort." Furthermore, the field of the invention relates to preservative systems that are broad ranged, and effective against not only bacterial, but also fungal sources of infection.

U..S. Pat. No. 4,758,595 (Ogunbiyi, et al.) discloses that polyhexamethylene biguanide (PHMB) and its water-soluble salts can fulfill minimal disinfection and be harmless to the eye and the lens, if used with a specific buffer, a surfactant, and in specific concentrations.

International Patent Publication No. WO 91/01763 discloses that solutions having very low concentrations of peroxide, i.e., from 0.01 to 0.5 percent more preferably 0.05 to 0.2 percent can provide disinfection without requiring neutralization. Use of the present invention greatly enhances the microbiocidal efficacy of peroxide in such low concentrations.

US-A-5,449,658 discloses biocidal compositions comprising polyhexamethylene biguanide and EDTA, and methods for treating commercial and recreational water. The growth of algae, fungi and pathogenic organisms in commercial and recreational waters, such as cooling towers, swimming pools and spas, may be controlled by adding to the water a primary sanitizing agent, preferably poly(hexamethylene biguanide) ("PHMB"), and a potentiating adjuvant comprising a calcium ion-chelating agent, preferably ethylenediamine tetraacetic acid ("EDTA"), in amounts such that the adjuvant renders the composition algicidal and fungicidal in water. The water may be further treated with a peroxy salt as a backup agent, preferably sodium perborate, or the calcium ion-chelating agent and peroxy salt may be combined as a shock treatment to water being treated with the primary sanitizing agent.

WO-A-92/11876 discloses a method for disinfecting a contact lens comprising contacting the lens with an isotonic aqueous solution comprising 0.6 to 2 weight percent tromethamine (preferably 0.8 to 1.5 percent) for a time sufficient to disinfect the lens. Other aspects include adding to the solution from 0.01 to 1 weight percent chelating agent (preferably disodium EDTA) and/or additional microbicide.

US-A-5,718,895 discloses enzymes with low isoelectric points for use in contact lens cleaning. Compositions containing an ophthalmically acceptable, modified enzyme exhibiting a low pl and methods involving the combined use of these compositions with a polymeric antimicrobial agent, are disclosed for the simultaneous cleaning and disinfecting of contact lens.

The present invention relates to improved solutions used in ophthalmic applications, where the improvement is increased preservative and anti-microbial efficacy. In particular it has been found that solutions comprising low levels of polyhexamethylene biguinide (PHMB) and a peroxide source, show increased preservative and anti-microbial activity over state of the art ophthalmic solutions.

### Summary of the Invention

The present invention relates to ophthalmic solutions that are broad ranged and effective in low concentrations relative to state of the art systems. In particular it has been found that ophthalmic solutions comprising 0.0100 to 0.0001 percent by weight of a peroxide producing agent and 0.1 to 500 parts per million by weight (PPM) of a cationic, polymeric preservative display an effective preservative capacity, and an increased capacity over state-of-the-art preservative systems.

The invention also relates to articles of manufacture that employ the solution in their operation. For instance, vials employed to store contact lenses for sale may be filled using the solution.

### Detailed Description of the Invention

The present invention relates to ophthalmic solutions that are broad ranged and effective in low concentrations relative to state of the art systems. In particular it has been found that ophthalmic solutions comprising comprising 0.0100 to 0.0001 percent by weight of a peroxide producing agent and 0.1 to 500 parts per million by weight (PPM) of a cationic, polymeric display improved effective preservative capacity, and greater capacity over state-of-the-art preservative systems.

Peroxide sources include hydrogen peroxide, sodium persulfate, sodium perborate decahydrate, sodium peroxide and urea peroxide. peracetic acid, an organic peroxy compound,

The cationic polymeric preservative includes polymeric biguanides such as polymeric hexamethylene biguanides (PHMB), and combinations thereof. Such cationic polymeric biguanides, and water-soluble salts thereof, having the following formula: 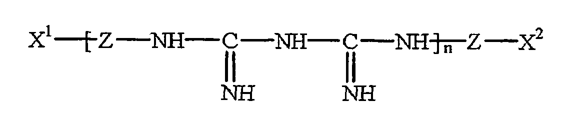 wherein Z is an organic divalent bridging group which may be the same or different throughout the polymer, n is on average at least 3, preferably on average 5 to 20, and X¹ and X² are

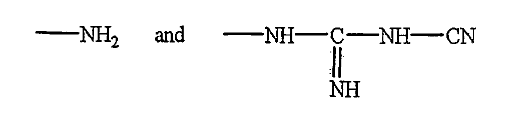

One group of water-soluble polymeric biguanides will have number average molecular weights of at least 1,000 and more preferably will have number average molecular weights from 1,000 to 50,000. Suitable water-soluble salts of the free bases include, but are not limited to hydrochloride, borate, acetate, gluconate, sulfonate, tartrate and citrate salts.

The above-disclosed biguanides and methods of preparation are described in the literature. For example, U.S. Pat. No. 3,428,576 describes the preparation of polymeric biguanides from a diamine and salts thereof and a diamine salt of dicyanimide.

Most preferred are the polymeric hexamethylene biguanides, commercially available, for example, as the hydrochloride salt from Zeneca (Wilmington, Del.) under the trademark CosmocilTM CQ. Such polymers and water-soluble salts are referred to as polyhexamethylene (PHMB) or polyaminoptopyl biguanide (PAPB). The term polyhexamethylene biguanide, as used herein, is meant to encompass one or more biguanides have the following formula: 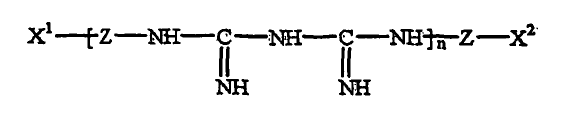 wherein Z, X¹ and X² are as defined above and n is from 1 to 500.

Depending on the manner in which the biguanides are prepared, the predominant compound falling within the above formula may have different X¹ and X² groups or the same groups, with lesser amounts of other compounds within the formula. Such compounds are known and are disclosed in U.S. Pat. No. 4,758,595 and British Patent 1,432,345. Preferably, the water-soluble salts are compounds where n has an average value of 2 to 15, most preferably 3 to 12.

In another embodiment, a polymeric biguanide is used in combination with a bis(biguanide) compound. Polymeric biguanides, in combination with bisbiguanides such as alexidine, are effective in concentrations as low as 0.00001 weight percent (0.1 ppm). It has also been found that the bactericidal activity of the solutions may be enhanced or the spectrum of activity broadened through the use of a combination of such polymeric biguanides with alexidine or similar biguanides.

An optional non-biguanide disinfectant/gennicide can be employed as a solution preservative, but it may also function to potentiate, complement or broaden the spectrum of microbiocidal activity of another germicide. This includes microbiocidally effective amounts of germicides which are compatible with and do not precipitate in the solution, in concentrations ranging from about 0.00001 to about 0.5 weight percent, and more preferably, from about 0.0001 to about 0.1 weight percent. Suitable complementary germicidal agents include, but are not limited to, quaternary ammonium compounds or polymers, thimerosal or other phenylmercuric salts, sorbic acid, alkyl triethanolamines, and mixtures thereof. Representative examples of the quaternary ammonium compounds are compositions comprised of benzalkonium halides or, for example, balanced mixtures of n-alkyl dimethyl benzyl ammonium chlorides. Other examples include polymeric quaternary ammonium salts used in ophthalmic applications such as poly[(dimethyliminio)-2-butene-1,4-diyl chloride], [4-tris(2-hydroxyethyl) ammonio]-2-butenyl-w-[tris(2-hydroxyethyl)ammonio]dichloride (chemical registry number 75345-27-6) generally available as polyquaternium 1 (r) from ONYX Corporation, or those described in U.S. Pat. No. 6,153,568.

The acid-addition salts of the germicides used in the present composition may be derived from an inorganic or organic acid. In most circumstances it is preferable that the salts be derived from an acid which is readily water soluble and which affords an anion which is suitable for human usage, for example a pharmaceutically-acceptable anion. Examples of such acids are hydrochloric, hydrobromic, phosphoric, sulphuric, acetic, D-gluconic, 2-pyrrolidino-5-carboxylic, methanesulphonic, carbonic, lactic and glutamic acids.

Peroxide sources may also be included in the formulations of the present invention and are exemplified by hydrogen peroxide, and such compounds , which provide an effective resultant amount of hydrogen peroxide, such as sodium perborate decahydrate, sodium peroxide, urea peroxide and peracetic acid, an organic peroxy compound.

The pH of the present solutions should be maintained within the range of 5.0 to 8.0, more preferably about 6.0 to 8.0, most preferably about 6.5 to 7.8. Suitable buffers may be added, such as boric acid, sodium borate, potassium citrate, citric acid, sodium bicarbonate, Bis-Tris Propane, TRIS, and various mixed phosphate buffers (including combinations of Na₂HPO₄, NaH₂PO₄ and KH₂PO₄) and mixtures thereof. Borate buffers are useful for enhancing the efficacy of PAPB. Generally, buffers will be used in amounts ranging from about 0.05 to 2.5 percent by weight, and preferably, from 0.1 to 1.5 percent.

The solutions of the present invention may further contain other additives including buffers, tonicity agents, demulcents, wetting agents, preservatives, sequestering agents (chelating agents), surface active agents, and enzymes.

Ophthalmologically acceptable chelating agents useful in the present invention include amino carboxylic acid compounds or water-soluble salts thereof, including ethylenediaminetetraacetic acid, nitrilotriacetic acid, diethylenetriamine pentaacetic acid, hydroxyethylethylenediaminetriacetic acid, 1,2-diaminocyclohexanetetraacetic acid, ethylene glycol bis (beta-aminoethyl ether) in N, N, N', N' tetraacetic acid (EGTA), aminodiacetic acid and hydroxyethylamino diacetic acid. These acids can be used in the form of their water soluble salts, particularly their alkali metal salts. Especially preferred chelating agents are the di-, tn- and tetra-sodium salts of ethylenediaminetetraacetic acid (EDTA), most preferably disodium EDTA (Disodium Edetate).

Other chelating agents such as citrates and polyphosphates can also be used in the present invention. The citrates which can be used in the present invention include citric acid and its mono-, di-, and tri-alkaline metal salts. The polyphosphates which can be used include pyrophosphates, triphosphates, tetraphosphates, trimetaphosphates, tetrametaphosphates, as well as more highly condensed phosphates in the form of the neutral or acidic alkali metal salts such as the sodium and potassium salts as well as the ammonium salt.

The solutions of the invention are compatible with both rigid gas permeable and hydrophilic contact lenses and other ophthalmic devices and instruments during storage, cleaning, wetting, soaking, rinsing and disinfection.

A typical aqueous solution of the present invention may contain additional ingredients which would not affect the basic and novel characteristics of the active ingredients described earlier, such as tonicity agents, surfactants and viscosity inducing agents, which may aid in either the lens cleaning or in providing lubrication to the eye. Suitable tonicity agents include sodium chloride, potassium chloride, glycerol or mixtures thereof The tonicity of the solution is typically adjusted to approximately 240-310 milliosmoles per kilogram solution (mOsm/kg) to render the solution compatible with ocular tissue and with hydrophilic contact lenses. In one embodiment, the solution contains 0.01 to 0.5 weight percent sodium chloride.

The solutions employed in the present invention may also include surfactants such as a polyoxyethylene-polyoxypropylene nonionic surfactant which, for example, can be selected from the group of commercially available surfactants having the name poloxamine or poloxamer, as adopted by The CTFA International Cosmetic Ingredient Dictionary. The poloxamine surfactants consist of a poly(oxypropylene)-poly(oxyethylene) adduct of ethylene diamine having a molecular weight from about 7,500 to about 27,000 wherein at least 40 weight percent of said adduct is poly(oxyethylene), has been found to be particularly advantageous for use in conditioning contact lenses when used in amounts from about 0.01 to about 15 weight percent. Such surfactants are available from BASF Wyandotte Corp., Wyandotte, Mich., under the registered trademark "Tetronic". The poloxamers are an analogous series of surfactants and are polyoxyethylene, polyoxypropylene block polymers available from BASF Wyandotte Corp., Parsippany, N.J. 07054 under the trademark "Pluronic".

The HLB of a surfactant is known to be a factor in determining the emulsification characteristics of a nonionic surfactant. In general, surfactants with lower HLB values are more lipophilic, while surfactants with higher HLB values are more hydrophilic. The HLB values of various poloxamines and poloxamers are provided by BASF Wyandotte Corp., Wyandotte, Mich. Preferably, the HLB of the surfactant in the present invention is at least 18, more preferably 18 to 32, based on values reported by BASF.

Additional compatible surfactants that are known to be useful in contact wetting or rewetting solutions can be used in the solutions of this invention. The surfactant should be soluble in the lens care solution and non-irritating to eye tissues. Satisfactory non-ionic surfactants include polyethylene glycol esters of fatty acids, e.g. coconut, polysorbate, polyoxyethylene or polyoxypropylene ethers of higher alkanes (C₁₂-C₁₈). Examples of the class include polysorbate 20 (available from ICI Americas Inc., Wilmington, Del. 19897 under the trademark Tween ® 20), polyoxyethylene (23) lauryl ether (Brij ® 35), polyoxyethylene (40) stearate (Myrj ® 52), polyoxyethylene (25) propylene glycol stearate (Atlas ® G 2612). Brij ® 35, Myrj ® 52 and Atlas ® G 2612 are trademarks of, and are commercially available from, ICI Americas Inc., Wilmington, Del. 19897.

Various other surfactants suitable for in the invention can be readily ascertained, in view of the foregoing description, from McCutcheon's Detergents and Emulsifiers, North American Edition, McCutcheon Division, MC Publishing Co., Glen Rock, N.J. 07452 and the CTFA International Cosmetic Ingredient Handbook, Published by The Cosmetic, Toiletry, and Fragrance Association, Washington, D.C. however, the preferred surfactants are commercially available surfactants sold under the trademark cremaphor ^{tm} by BASF and which are polyoxyethoxylated castor oils.

Suitable viscosity inducing agents can include lecithin or the cellulose derivatives such as hydroxymethylcellulose, hydroxypropylcellulose and methylcellulose in amounts similar to those for surfactants, above.

### EXAMPLE 1

A set of aqueous solutions containing Pluronic F127 (0.1 %) and glycerin (2%) was prepared the pH was adjusted to pH 7.65. Polyhexamethylene biquanide (PHMB) was added to half of this solution to yield a final concentration of 1 ppm. Another set of aqueous solutions containing hydrogen peroxide (60 ppm), Pluronic ® F127 (0.1 %) and glycerin (2.3%) was prepared the pH was adjusted to pH 7.35. Polyhexamethylene biquanide (PHMB) was added to half of this solution to yield a final concentration of 1 ppm.

Each of these solutions were tested for their activity against *S. aureus* and *C. albicans.* The data are summarized in the following table.

| | *S. **aureus*** | ***C. albicans*** |
|---|---|---|
| **Formulation** | **4 hours** | **4 hours** |
| No preservative | 0.05 | -0.09 |
| PHMB | 4.03 | 2.40 |
| | | |
| Hydrogen peroxide | 1.95 | 1.06 |
| Hydrogen peroxide, PHMB | > 4.73 | 3.08 |
| Marketed Product 1 * | > 4.73 | 0.54 |
| Marketed Product 2 ** | > 4.73 | 2.57 |

| | | |
|---|---|---|
| * marketed product 1 having the general composition: A sterile isotonic aqueous solution containing sodium chloride, polyoxyethylene polyoxypropylene block copolymer, sodium phosphate dibasic, sodium phosphate monobasic, and preserved with edetate disodium dihydrate 0.025% and polyhexanide 0.0001 %. ** marketed product 2 having the general composition: A sterile, isotonic solution that contains HYDRANATE®(hydroxyalkylphosphonate), boric acid, edetate disodium, poloxamine, sodium borate and sodium chloride; preserved with DYMED® (polyaminopropyl biquanide) 0.0001%. | | |

The results demonstrate the improved efficacy of the polyhexamethylene biquanide - hydrogen peroxide combination against S. aureus and C. albicans. The effectiveness was superior to that found in either commercially marketed products.

### Example 2 PHMB - Peroxide

Formulations were prepared by dissolving L-histidine or Bis-Tris Propane in water. The pH of the solutions were adjusted to 7.3 with 1 N hydrochloric acid. Hydrogen peroxide, Dequest ® 2010 and polyhexamethylenebiguanide HCI (PHMB) were added to these solutions. The formulations were diluted to volume with water. Each of these solutions were tested for their activity against *C. albicans* (ATCC 10231) following a two hour exposure. The activity is expressed as a log reduction from the initial inoculum. The compositions, concentrations and activity of each of the solutions are summarized in the following table.

| **Log** | **Preservative** | **Buffer** | **Hydrogen** | **Dequest** |
|---|---|---|---|---|
| **Reduction** | | | **Peroxide** | **2010** |
| 1.59 | Bis-Tris Propane 0.2% | none | none | 0.006% |
| 2.05 | Bis-Tris Propane 0.2% | none | 0.006% | 0.006% |
| | | | | |
| 1.25 | L-histdine 0.2% | none | none | 0.006% |
| 1.85 | L-histdine 0.2% | none | 0.006% | 0.006% |

The results demonstrate the improved antifungal efficacy of the polyhexamethylene biguanide - hydrogen peroxide combination against C. *albicans.*

### Example 3

### PHMB -

Formulations were prepared by dissolving L-histidine, Bis-Tris Propane, or Tricine in water. The pH of the solutions were adjusted to 7.3 with 1 N hydrochloric acid. Glycerin, hydrogen peroxide, Dequest 2010 and polyhexamethylenebiguanide HCl (PHMB) were added to these solutions. The formulations were diluted to volume with water. Each of these solutions were tested for their activity against C. *albicans* (ATCC 10231) following a two hour exposure. The activity is expressed as a log reduction from the initial inoculum. The compositions, concentrations and activity of each of the solutions are summarized in the following table.

| **Log Reduction** | **Preservative** | **Buffer** | **Glycerin** | **Hydrogen Peroxide** | **Dequest 2010** |
|---|---|---|---|---|---|
| 1.60 | PHMB 0.0001 % | L-Histidine 0.2% | none | none | none |
| 2.38 | PHMB 0.0001 % | L-Histidine 0.2% | none | 0.006% | none |
| | | | | | |
| 1.27 | PHMB 0.0001 % | L-Histidine 0.2% | none | none | 0.006% |
| 2.25 | PHMB 0.0001 % | L-Histidine 0.2% | none | 0.006% | 0.006% |
| | | | | | |
| 1.08 | PHMB 0.0001% | L-Histidine 0.2% | none | none | 0.003% |
| 2.04 | PHMB 0.0001% | L-Histidine 0.2% | none | 0.006% | 0.003% |
| | | | | | |
| 1.57 | PHMB 0.0001% | L-Histidine 0.2% | 0.50% | none | none |
| 2.15 | PHMB 0.0001 % | L-Histidine 0.2% | 0.50% | 0.006% | none |
| | | | | | |
| 1.25 | PHMB 0.0001 % | L-Histidine 0.2% | 0.50% | none | 0.006% |
| 2.04 | PHMB 0.0001 % | L-Histidine 0.2% | 0.50% | 0.006% | 0.006% |
| | | | | | |
| 1.08 | PHMB 0.0001% | L-Histidine 0.2% | 0.50% | none | 0.003% |
| 1.93 | PHMB 0.0001 % | L-Histidine 0.2% | 0.50% | 0.006% | 0.003% |
| | | | | | |
| 2.80 | PHMB 0.0001% | Bis-Tris Propane 0.2% | none | none | none |
| 3.69 | PHMB 0.0001% | Bis-Tris Propane 0.2% | none | 0.006% | none |
| | | | | | |
| 2.20 | PHMB 0.0001% | Bis-Tris Propane 0.2% | none | none | 0.006% |
| 3.18 | PHMB 0.0001% | Bis-Tris Propane 0.2% | none | 0.006% | 0.006% |
| | | | | | |
| 2.18 | PHMB 0.0001% | Bis-Tris Propane 0.2% | none | none | 0.003% |
| 3.05 | PHMB 0.0001% | Bis-Tris Propane 0.2% | none | 0.006% | 0.003% |
| | | | | | |
| 2.78 | PHMB 0.0001% | Bis-Tris Propane 0.2% | 0.50% | none | none |
| 3.32 | PHMB 0.0001% | Bis-Tris Propane 0.2% | 0.50% | 0.006% | none |
| | | | | | |
| 2.29 | PHMB 0.0001% | Bis-Tris Propane 0.2% | 0.50% | none | 0.006% |
| 3.29 | PHMB 0.0001% | Bis-Tris Propane 0.2% | 0.50% | 0.006% | 0.006% |
| | | | | | |
| 2.13 | PHMB 0.0001% | Bis-Tris Propane 0.2% | 0.50% | none | 0.003% |
| 3.31 | PHMB 0.0001% | Bis-Tris Propane 0.2% | 0.50% | 0.006% | 0.003% |
| | | | | | |
| 1.64 | PHMB 0.0001% | Tricine 0.2% | none | none | none |
| 2.05 | PHMB 0.0001% | Tricine 0.2% | none | 0.006% | none |
| | | | | | |
| 1.16 | PHMB 0.0001% | Tricine 0.2% | none | none | 0.006% |
| 1.76 | PHMB 0.0001% | Tricine 0.2% | none | 0.006% | 0.006% |
| | | | | | |
| 1.17 | PHMB 0.0001% | Tricine 0.2% | none | none | 0.003% |
| 1.78 | PHMB 0.0001% | Tricine 0.2% | none | 0.006% | 0.003% |

The results demonstrate the improved antifungal against *C. albicans* in each paired formulation, when 0.006% hydrogen peroxide is added. The data demonstrates that the increased activity is independent on the presence of Dequest 2010.

### Example 4

### PHMB - Peroxide

Formulations were prepared by dissolving Tricine, Citiric Acid, Bicine, L-histidine,Glycine, or Lysine in water. The pH of the solutions were adjusted to 7.3 with 1 N hydrochloric acid. Hydrogen peroxide, Dequest ® 2010 and polyhexamethylenebiguanide HCl (PHMB) were added to these solutions. The formulations were diluted to volume with water. Each of these solutions were tested for their activity against C. *albicans* (ATCC 10231) following a two hour exposure. The activity is expressed as a log reduction from the initial inoculum. The compositions, concentrations and activity of each of the solutions are summarized in the following table.

| **Log Reduction** | **Preservative** | **Buffer** | **Hydrogen Peroxide** | **Dequest 2010** |
|---|---|---|---|---|
| 1.90 | PHMB 0.0001% | Tricine 0.2% | none | none |
| 2.09 | PHMB 0.000 1 % | Tricine 0.2% | 0.006% | 0.003% |
| | | | | |
| 0.25 | PHMB 0.0001% | Citric Acid 0.2% | none | none |
| 0.70 | PHMB 0.0001 % | Citric Acid 0.2% | 0.006% | 0.003% |
| | | | | |
| 2.01 | PHMB 0.0001 % | Bicine 0.2% | none | none |
| 2.47 | PHMB 0.0001% | Bicine 0.2% | 0.006% | 0.003% |
| | | | | |
| 2.01 | PHMB 0.0001% | Histidine 0.2% | none | none |
| 2.42 | PHMB 0.0001 % | Histidine 0.2% | 0.006% | 0.003% |
| | | | | |
| 1.94 | PHMB 0.0001 % | Glycine 0.2% | none | none |
| 2.89 | PHMB 0.0001% | Glycine 0.2% | 0.006% | 0.003% |
| | | | | |
| 2.69 | PHMB 0.0001% | Lysine 0.2% | none | none |
| 2.84 | PHMB 0.0001% | Lysine 0.2% | 0.006% | 0.003% |

The results demonstrate the improved antifungal efficacy of the polyhexamethylene biguanide - hydrogen peroxide combination.

### Example 5

### PHMB - Peroxide

Formulations were prepared by dissolving Bis-Tris Propane, L-histidine, or Tricine in water. The pH of the solutions were adjusted to 7.3 with 1 N hydrochloric acid. The tonicity agent, hydrogen peroxide, Dequest 2010 and polyhexamethylenebiguanide HCl (PHMB) were added to these solutions. The formulations were diluted to volume with water. Each of these solutions were tested for their activity against *C. albicans* (ATCC 10231) following a two hour exposure. The activity is expressed as a log reduction from the initial inoculum. The compositions, concentrations and activity of each of the solutions are summarized in the following table.

| **Log** | | | | | **Hydrogen** | **Deques** |
|---|---|---|---|---|---|---|
| **Reduction** | **Preservative** | **Buffer** | **Tonicity Agent** | **Wetting Agent** | **Peroxide** | **2010** |
| 3.85 | PHMB 0.0001% | Bis-Tris Propane | none | Cremophor RH 40 | none | none |
| | | 0.2% | | | | |
| 4.70 | PHMB 0.0001% | Bis-Tris Propane | none | Cremophor RH 40 | 0.006% | 0.003% |
| | | 0.2% | | | | |
| 2.42 | PHMB 0.0001% | L-Histidine 0.2% | none | Cremophor RH 40 | none | none |
| 3.34 | PHMB 0.0001% | L-Histidine 0.2% | none | Cremophor RH 40 | 0.006% | 0.003% |
| 2.17 | PHMB 0.0001% | Tricine | none | Cremophor RH 40 | none | none |
| 2.69 | PHMB 0.0001% | Tricine | none | Cremophor RH 40 | 0.006% | 0.003% |
| 3.70 | PHMB 0.0001% | Bis-Tris Propane | glycerin 3% | Cremophor RH 40 | none | none |
| | | 0.2% | | | | |
| 4.40 | PHMB 0.0001% | Bis-Tris Propane | glycerin 3% | Cremophor RH 40 | 0.006% | 0.003% |
| | | 0.2% | | | | |
| 2.19 | PHMB 0.0001% | L-Histidine 0.2% | glycerin 3% | Cremophor RH 40 | none | none |
| 2.94 | PHMB 0.0001% | L-Histidine 0.2% | glycerin 3% | Cremophor RH 40 | 0.006% | 0.003% |
| 2.19 | PHMB | Tricine | glycerin 3% | Cremophor RH | none | none |
| | 0.0001% | | | 40 | | |
| 2.45 | PHMB 0.0001% | Tricine | glycerin 3% | Cremophor RH 40 | 0.006% | 0.003% |
| 2.19 | PHMB 0.0001% | L-Histidine 0.2% | propylene glycol | Cremophor RH 40 | none | none |
| | | | 3% | | | |
| 2.95 | PHMB 0.0001% | L-Histidine 0.2% | propylene glycol | Cremophor RH 40 | 0.006% | 0.003% |
| | | | 3% | | | |
| 4.40 | PHMB 0.0001% | Bis-Tris Propane | sorbitol 5% | Cremophor RH 40 | none | none |
| | | 0.2% | | | | |
| 4.70 | PHMB 0.0001% | Bis-Tris Propane | sorbitol 5% | Cremophor RH 40 | 0.006% | 0.003% |
| | | 0.2% | | | | |
| 3.36 | PHMB 0.0001% | L-Histidine 0.2% | sorbitol 5% | Cremophor RH 40 | none | none |
| 3.92 | PHMB 0.0001% | L-Histidine 0.2% | sorbitol 5% | Cremophor RH 40 | 0.006% | 0.003% |
| 2.54 | PHMB 0.0001% | L-Histidine 0.2% | inositol 5% | Cremophor RH 40 | none | none |
| 3.08 | PHMB 0.0001% | L-Histidine 0.2% | inositol 5% | Cremophor RH 40 | 0.006% | 0.003% |

The data shows that the addition of 0.006% hydrogen peroxide to polyhexamethylene biguanide provides increased antifungal ctivity against C. *albicans*. Consistent results were found in the presence of Cremophor RH40 with histidine, tricine, Bis-Tris Propane, glycerin, propylene glycol, and soribitol.

## Claims

1. An ophthalmic solution comprising 0.01 to 0.0001 percent by weight of a peroxide producing agent chosen from the group consisting of hydrogen peroxide, sodium peroxide, urea peroxide and peracetic acid, and 0.1 to 500 parts per million of a cationic, polymeric preservative.

2. The solution of claim 1 wherein said cationic, polymeric preservative represented by the chemical formula: 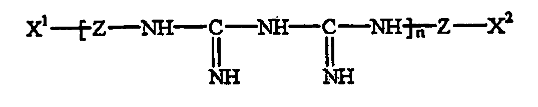 wherein Z is an organic divalent bridging group which may be the same or different throughout the polymer, n is on average at least 3, and X¹ and X² are chosen from the group consisting of:

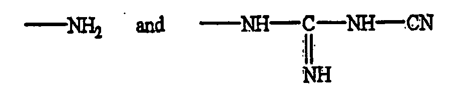

3. The solution of Claim 2 wherein n is equal to on average 5 to 20.

4. The solution of Claim 1 that further comprises 0.05 to 2.5 percent by weight of a buffer chosen from the group consisting of boric acid, sodium borate, potassium citrate, citric acid, sodium bicarbonate, TRIS, Na₂HPO₄, NaH₂PO₄ and KH₂PO₄, and mixtures thereof.

5. The solution of Claim 4 that further comprises a surfactant.

6. A contact lens vial comprising:
a vial;
a contact-lens; and a sufficient amount of a solution to immerse said contact lens, wherein said solution comprises 0.01 to 0.0001 percent by weight of a peroxide producing agent and 0.1 to 500 ppm of a cationic, polymeric preservative.

## Patentansprüche

1. Ophthalmische Lösung, die aus Folgendem besteht:
0,01 bis 0,0001 Gew.-% eines Peroxid produzierenden Mittels, welches aus der Gruppe ausgewählt ist, die aus Wasserstoffperoxid, Natriumperoxid, Carbamidperoxid bzw. Ureaperoxid und Peroxyessigsäure besteht, und
0,1 bis 500 ppm eines kationischen, polymeren Konservierungsmittels.

2. Lösung nach Anspruch 1, in der das kationische, polymere Konservierungsmittel, durch folgende chemische Formel repräsentiert wird: 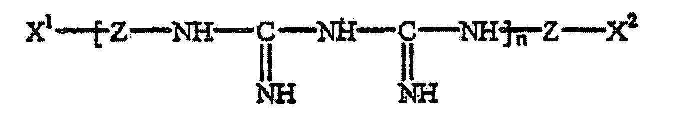 wobei Z ein organisches bivalentes Brückenglied ist, welche im gesamten Polymer gleich oder unterschiedlich ist, wobei n durchschnittlich mindestens 3 ist, und X¹ und X² ausgewählt sind aus der Gruppe, die aus 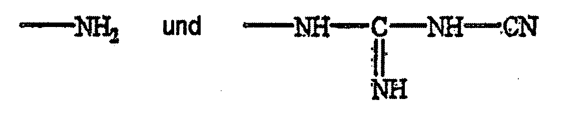 besteht.

3. Lösung nach Anspruch 2, wobei n durchschnittlich gleich 5 bis 20 ist.

4. Lösung nach Anspruch 1, welche ferner Folgendes aufweist: 0,05 bis 2,5 Gewichts-% eines Puffers, der aus der Gruppe ausgewählt ist, die aus Borsäure, Natriumborat, Kaliumcitrat, Zitronensäure, Natriumhydrogencarbonat, TRIS, Na₂HP₄, NAH₂PO₄ und KH₂PO₄ und Mischungen daraus besteht.

5. Lösung nach Anspruch 4, welche ferner ein Benetzungsmittel bzw. eine grenzflächenaktive Substanz aufweist.

6. Ein Kontaktlinsenbehälter, welcher Folgendes aufweist:
einen Behälter;
eine Kontaktlinse; und
eine ausreichende Menge einer Lösung, um die Kontaktlinse darin unterzutauchen, wobei die Lösung 0,01 bis 0,0001 Gewichts-% eines Peroxid produzierenden Mittels und 0,1 bis 500 ppm eines kationischen, polymeren Konservierungsmittels aufweist.

## Revendications

1. Solution ophtalmique comprenant de 0,01 à 0,0001 % en poids d'un agent de production de peroxyde choisi dans le groupe comprenant le peroxyde d'hydrogène, le peroxyde de sodium, le peroxyde d'urée et l'acide peracétique, et 0,1 à 500 parties par million d'un conservateur polymère cationique.

2. Solution selon la revendication 1, dans laquelle le conservateur polymère cationique est représenté par la formule chimique : 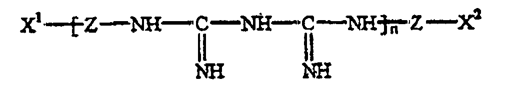 où Z est un groupe de pontage divalent organique qui peut être le même dans tout le polymère ou être différent, n est en moyenne au moins égal à 3, et X¹ et X² sont choisis dans le groupe comprenant :

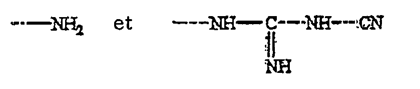

3. Solution selon la revendication 2, dans laquelle n est en moyenne compris entre 5 et 20.

4. Solution selon la revendication 1, comprenant en outre de 0,05 à 2,5 % en poids d'une solution tampon choisie dans le groupe comprenant l'acide borique, le borate de sodium, le citrate de potassium, l'acide citrique, le bicarbonate de sodium, le TRIS, le Na₂HPO₄, le NaH₂PO₄, et le KH₂PO₄, et leurs mélanges.

5. Solution selon la revendication 4, comprenant en outre un agent de surface.

6. Fiole pour lentille de contact comprenant :
une fiole ;
une lentille de contact ; et
une solution en quantité suffisante pour immerger la lentille de contact, la solution comprenant entre 0,01 et 0,0001 % en poids d'un agent de production de peroxyde et entre 0,1 et 500 ppm d'un conservateur polymère cationique.
